# EUROPEAN PATENT SPECIFICATION

(11) **EP 0 105 872 B1**
(45) Date of publication and mention of the grant of the patent: **03.08.1988**
(21) Application number: 82901361.4
(22) Date of filing: 23.04.1982
(51) Int. Cl.: A61K 7/16, A61K 7/22, A61K 7/32, C07C 87/50, C07C 93/18, C07C 93/193

(54) **NOVEL COSMETIC COMPOSITIONS CONTAINING PRENYLAMINE AND A PROCESS FOR THEIR PREPARATION**
KOSMETISCHE ZUSAMMENSETZUNGEN ENTHALTEND PHENYLAMIN UND DEREN HERSTELLUNG
NOUVELLES COMPOSITIONS COSMETIQUES CONTENANT DE LA PRENYLAMINE ET LEUR PROCEDE DE PREPARATION

(43) Date of publication of application: 25.04.1984
(73) Proprietor: CAOLA KOZMETIKAI ES HAZTARTÄSVEGYIPARI VALLALAT, 1113 Budapest (HU)
(72) Inventor: SZATLOCZKY, Ernö, H-1025 Budapest (HU); BOROS, Kálmán, H-1138 Budapest (HU); CSAKVARI, Gábor, H-1118 Budapest (HU); GYARMATI, István, H-1145 Budapest (HU); ZALAY, László, H-1093 Budapest (HU)
(74) Representative: von Füner, Alexander, Dr.
(86) International application number: HU8200018
(87) International publication number: WO8303755

(56) References cited:
- FR-M- 553
- US-A- 3 953 599
- US-A- 4 093 711
- US-A- 4 098 880
- US-A- 4 100 270
- US-A- 4 102 993
- US-A- 4 224 309
- US-A- 4 224 309
- M.D. Mashkovsky "Lekarstvennye sredstva", Part II, 1977, Meditsina (Moscow), 403

## Description

The invention relates to the use of 3,3-diphenylpropyl-1-methylphenethylamine (prenilamine) or one of the acid addition salts in cosmetic preparations, in particular toothpastes, mouthwashes, deodorants, creams, shaving creams, soaps, foam baths and shampoos. The new cosmetics are preferably suitable in the body care of persons with greasy and sensitive skin, for the skin care of persons inclined to allergy and for the prevention of certain oral disorders.

In accordance with the conventional view, the cosmetics are used to beautify the skin, and are intended to improve the aesthetic, pleasant properties of the skin. The modern view takes the term "cosmetics" to mean substances which are brought into contact with the various parts of the human body (skin, hair, nails, lips, teeth, genitals, mucosa) in particular to protect them, to maintain them in good condition, to enhance their appearance and their fragrance and to prevent body odour.

The purchasers of cosmetics expect over higher standards, and the demands made upon cosmetic preparations are constantly increasing. The preparation of propylactically active cosmetics is constantly widening its scope. Prophylactically active cosmetics include for example the fluorine-containing [sic] toothpastes which prevent caries, the deoderants which contain bacteriostatics, biologically active substances and creams containing vitamins and allergen-free cosmetics.

In the cosmetic industry the preparation of products of this nature is subject to considerable difficulties, for example the microbiological purity of modern, highly effective preparations. The cosmetic industry is engaged in a vigorous struggle, on the one hand with the aim for its preparations to reach the consumer with the minimum number of germs and properly stabilized, and on the other hand with the supervising health authorities in order to increase the range of the antibacterial substances which can be used against the increasingly more resistant strains of bacteria, are subject to official approval and are available in only limited numbers. In practice, in order to preserve and stabilize creams antibacterially active substances are used, for example methyl paraben (p-hydroxybenzoic acid methylester) or propyl paraben (p-hydroxybenzoic acid propylester).

The specialists in the cosmetic industry have recently made the disturbing discovery that the range of usable antibacterially active substances is insufficient. The constant application has resulted in the development of resistant strains. The increases in the concentration of the antibacterial substances permitted for cosmetic purposes has led to many cases to detrimental dermatological symptoms, which have completely overshadowed the original effect of the cosmetic. In other cases, for example in the case of hexachlorophene (3,3',4,4',6,6'-hexachloro-2,2'-methylene-diphenoi), embryotoxic phenomena have been observed.

The preparation of cosmetics with the minimum number of germs is consequently an important problem which has not been satisfactorily solved until now. The microbiological purity of the cosmetic preparations is particularly important when the cosmetic comes into contact with the chronically, latently sick parts of the body of the user during its application. The areas most at risk are the following: the oral cavity, the eye and its surrounding area, the genitals, the axilla, the area between the toes and the entire body surface of babies.

Experience has shown that the preserving substances used at present cannot be regarded as remotely satisfactory. In many cases it can be assumed that acute inflammation of the oral mucosa, conjunctivitis or allergic symptoms have resulted from an inadequately sterile cosmetic.

Cosmetics are produced for healthy persons. In some cases, however, the purchaser is seeking a cosmetic for a syndrome which from the outset is a matter for the dermatologist. In such a case, the cosmetic which is insufficiently germ-free and contains no prophylactics harms more than it helps the sensitive skin which is already diseased.

Even the pure cosmetic can harm the skin. Generally the skin then becomes inflamed at the site of application. Contact dermatitis begins at the point of application with pruritus, erythema, oedematous swelling, blisters are formed, and even the pigmentation of the skin can be adversely affected. This syndrome can spread over the entire body.

Contact dermatitis is generally characteristic of persons with allergic skin. The allergenic substance causing the allergy can occur in the aromatic composition of the cosmetic, but the surface-active substance or the disinfectant itself, dyes, base materials of unsuitable quality, lanolin, toxins of bacteria possibly contained in the cosmetic can also be allergens. In these cases, conjunctivitis of the eye, inflamed diseases of the mucosa of the mouth and genitals, swelling of the lips and pigment disorders of the skin on account of the action of light can rapidly occur as secondary processes.

Today, the consumer already expects suitable cosmetics to be available for types of disease which appear as blemisches but which in fact represent chronic dermatological cases. Such types of disease are: greasy and dry seborrhoea, pimples in youth, comedones, erisypelas, excessive dryness or greasiness of the facial skin, excessive perspiration, hypertrichosis, in certain respects also diseases of the hair and nails, fungal diseases and not least psoriasis, and in additional from the gynaecological point of view discharge and pruritus. The consumer also expects toothpastes and mouthwashes to guard against caries. At present there are no specific medicaments which can be used without risk to combat the diseases enumerated.

The problems outlined above being at present to the main difficulties with which the cosmetic industry has to contend, since there is no substance presently available which would meet the complex cosmetic requirements.

It is known that prenilamine lactate and prenilamine fumarate have not only a weak β-sympatholytic action, but for treating many gastroenterological diseases, for example the so-called dumping syndrome, fermentation dyspepsia, chronic diarrhoea, lactose intolerance, food intolerance and skin changes linked to dyspepsia (Szatlóczky and lrsay: Medicus Universalis, 2, 17-19, 1969; Szatlóczky and Bartha: Dtsch. Med. Wschr. (German Medical Journal) 97, 1125, 1972; Szatlóczky, Racz, Czákvári, Zalay and Temesvary: Travelling Assembly VIII of the Hungarian Society for Allergology and Clinical Immunology, Budapest, 19th to 21st April 1978, Abstract of Lectures p. 24; 21st Plenary Session of the Hungarian Oynaecological Society, Debrecen, 8th to 11th November 1981, Abstracts p. 28).

The use of prenilamine in medicaments is known from FR-M-553. In the book "kapcTBeHHWe cpeAcTBa" (Lekarstvennye sredstva), M. B. Mashkovsky, Moscow, Medicine Publishing House, 1977, p. 403, it is stated that prenilamine has a sympatholytic action, lowers the resistance of peripheral vessels and reduces the arterial pressure to a substantial extent. It also has a coronary-windening effect and improves metabolic processes in the myocardium. It is used in the case of chronic coronary disorders and to prevent cardiac infarct.

Clinical experience has shown that prenilamine in the gastric and intestinal tract prevents absorption and also has a bacteriostatic or bactericidal effect.

When extending the experiments to specific diseases and cosmetic disorders of the skin, it was surprisingly found that the prenilamine or the cosmetic preparations thereof containing acid addition salts are pre-eminently suitable for reducing the extreme sensitivity of the human skin and mucosa, for normalizing the skin and making it germ-free, for deodorizing the products of the sudoriferous glands, for calming the pruritus of the skin, for promoting the growth of hair and in individual cases for preventing the greying of the hair.

The object of the invention is the use of 3,3-diphenylpropyl-1-methylphenethylamine or one of its acid addition salts in cosmetic preparations.

3,3-diphenylpropyl-1-methylphenethylamine lactate or 3,3-diphenylpropyl-1-methylphenethylamine fumarate is preferably'used.

The cosmetic preparations according to the invention may be for example in the form of toothpaste, mouthwash, oral spray, cream, deodorants, shaving cream, soap, foam bath or shampoo and generally contain 0.05-8.0% by weight of 3,3-diphenylpropyl-1-methylphenethylamine or an acid addition salt thereof, 20-99% by weight of solvent or solvent mixture, 0-70% by weight of washing active substances and/or emulsifiers, 0-20% by weight of cosmetic additives and 0-35% by weight of fillers, and, where appropriate, in addition to the preparation poured into aerosol canisters, a propellent can also be present and in this case the weight ratio between the preparation to be atomized and the propellent lies-between 1:1 and 20:1.

A preferred group of the preparations according to the invention is formed by the oral care agents, e.g. toothpaste, tooth gel, mouthwash or oral spray. Toothpaste which in general contains 0.05-8.0% by weight, preferbaly 0.5-2.0% by weight, of prenilamine or an acid addition salt thereof is particularly advantageous.

Another preferred group of the cosmetic preparations according to the invention is formed by the deodorants which, in addition to the components usual in deodorants, generally contain 0.05-8% by weight, preferably 0.5-2.0% by weight of prenilamine or an acid addition salt thereof. The deodorants according to the invention can be aerosol deodorants, solutions or sticks.

Preferred solvents are: water, lower aliphatic alcohols, e.g. ethanol, higher aliphatic alcohols, e.g. cetyl alcohol, octyl dodecanol or oleyl alcohol, fatty acids, e.g. stearic acid, fatty acid alkali salts, saturated or unsaturated fatty acid esters, e.g. myristic acid propyl esters or oleic acid fatty alcohol esters, polyatomic alcohols, e.g. glycerine or polyglycol ether, the mono-, di- or tri-fatty acid esters of glycerine, paraffin oil or mixtures of the substances enumerated.

The washing active substances can be the substances commonly used in cosmetic preparations, e.g. sodium lauryl sulphate, sodium lauryl ether sulphate or fatty alcohol ether sulphate. These substances can be used for example in shampoos. Polyoxyethylenesorbitan -monostearate for example is possible as an emulsifier. Emulsifiers are necessary mainly in creams and body care lotions.

The cosmetic additives are the substances commonly used in cosmetic preparations, for example aromatics and flavouring agents, sweetening agents-advantageously benzosulphimide (3-oxo-2,3-dihydro-1,2-benzisothiazol-1,1-dioxide), sorbit or xylite-dyes and pigments, for example titanium dioxide fluorine compounds, for example sodium fluoride or sodium monofluorophosphate, coconut oil acid diethanol amide, potassium carbonate, lanolin, silicone oil, biologically active substances such as cholesterol, vitamins etc.

The fillers may be for examplke dicalcium phosphate, dispersed silicic acid, mainly in the preparation of toothpaste.

If the cosmetic preparation is to be used in the form of an aerosol, it will be poured into aerosol canisters in the course of manufacture, and in addition the quantity of one of the commonly used propellants required for atomization.

Prenilamine and its acid addition salts do not dissolve well in water and are therefore per se absorbed by the skin only with difficulty. It is thus a source of surprise to the person skilled in the art that solvents such as for example glycerine, cetyl alcohol, polyethylene glycol, polypropylene glycol, myristic acid isopropyl ester or glycerine monostearate, which promote the hydration of the skin, ensure the penetration of prenilamine and the salts thereof into the upper dermal layers. The transfollicular absorption of emulsions prepared with emulsifiers takes place somewhat more slowly but still sufficiently quickly. Prenilamine or its salt is absorbed by the skin particularly rapidly when it is applied as an aerosol. A particularly preferred form of the cosmetics according to the invention is thus constituted by the types of preparation which can be produced in the form of aerosols.

Prenilamine or its acid addition salt present in a dissolved form in the cosmetic preparations according to the invention at the same time makes the preparation stable, i.e. it is generally not necessary to add a chemical preservative which only increases the sensitivity of the skin.

The cosmetic preparations according to the invention can contain the biologically active substances which are customary in the cosmetic industry and with which the prenilamine where appropriate exerts a synergistic effect.

The new cosmetic preparations according to the invention may belong for example to the following types of preparation: tooth cream, mouthwash, deodorants, intimate deodorants, cream, protective cream, soaps, face lotions, facial milk, body care lotions, hair care agents, pedicure agents, shampoo, foam bath, anti-perspirant agents, light-protection agents, shaving cream, articles of baby and child care.

The agents according to the invention are packed in conventional manner, for example in tubes, pots, glass jars, plastics jars, plastics foil etc. A particularly preferred form is the solution which can be converted by atomization into an aerosol and is provided with a propellent known per se.

The cosmetic preparations according to the invention are produced by mixing their components. In general 0.05-8.0% by weight of prenilamine or prenilamine acid addition salt is dissolved in a solvent or mixture of sovlents, where desired additional solvent is added up to a maximum or a quantity of 20-99% by weight, the solution is mixed with 0-70% by weight of washing active substances and/or emulsifiers, 0-20% by weight of cosmetic additives and 0-35% by weight of fillers, and the mixture obtained is poured into the vessels customary for packing cosmetic preparations. In the production of aerosol preparations the weight ratio between the preparation to be atomized and the propellent lies within the range of 1:1 and 20:1.

The cosmetic agents according to the invention can advantageously be used primarily with persons whose skin is particularly sensitive or greasy or susceptible to allergic disorders or exhibits allergic symptoms. The preparations according to the invention properly disinfect the skin or mucosa respectively and reduce pruritus, i.e. they calm the skin. These properties are advantageous in cases of chronic irritation of the mucosa. Persons with very sensitive skin can apply the preparations according to the invention subsequently to the foam baths or sunscreen agents which contain surfactants and therefore dry the skin. The application of the hair care agent according to the invention combats scaly scalps and enhances the growth and pigmentation of the hair. The deodorant according to the invention deodorizes the secretions of the sudatory and sebaceous glands and normalizes their functioning.

The functioning mechanism of the preparations according to the invention has not yet been clarified, but it can be assumed that they inhibit inter alia the release of histamine and histamine-like substances in the skin and act upon the symptoms caused by overproduction of PGFa.

The prenilamine contained in the cosmetic preparations according to the invention inhibits the growth of Gram-positive and Gram-negative bacteria, yeasts and fungi. When compared with other preservatives extensively used in cosmetics, its action is extremely favourable, and its toxicity is low. The activity extends over a wide pH range. The minimum inhibition concentration (MIC) of some known preservatives and their optimum pH range is set out in the following table. The trivial names stand for the following compounds:

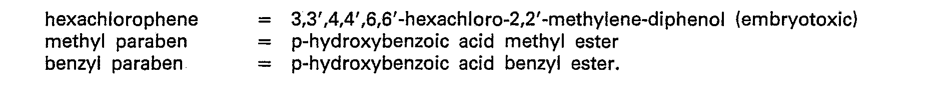

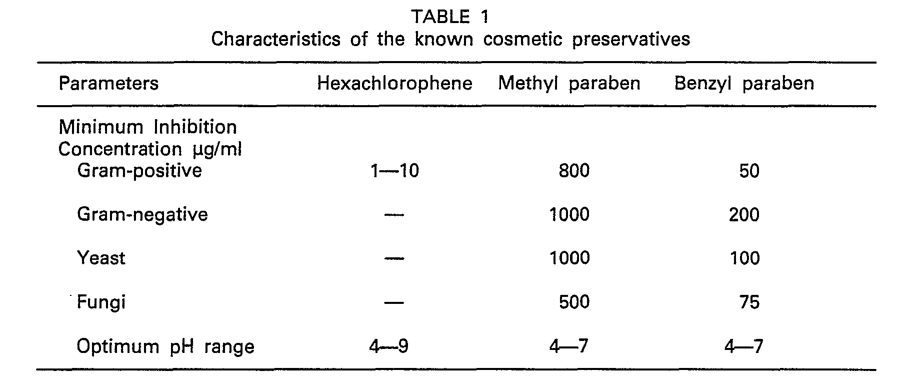

The minimum inhibition concentrations of prenilamine for some microorganisms are indicated in the following Table 2.

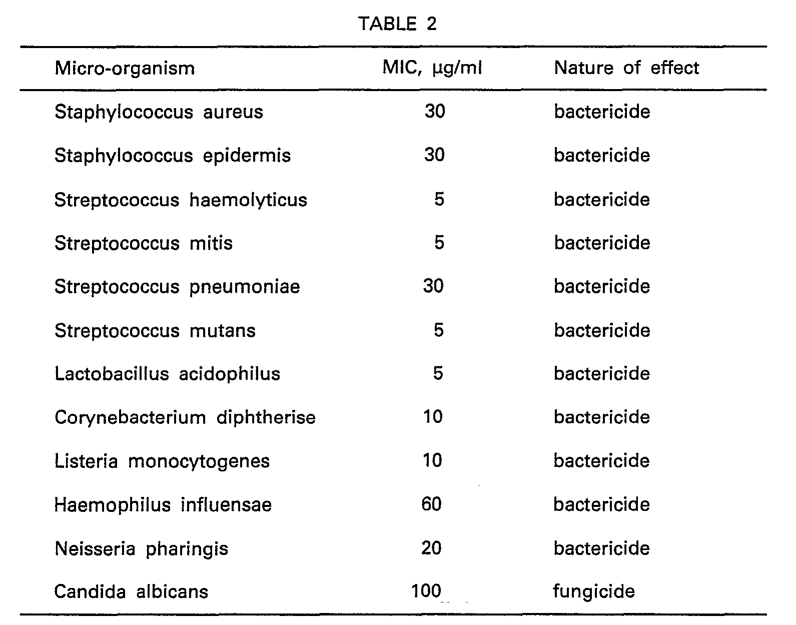

The cosmetic preparations containing 0.05-0.25% by weight of prenilamine have already a bacteriostatic effect. In the case of the preparations containing 0.25@0.9% by weight of prenilamine a bactericidal action may also be observed. With the cosmetic preparations containing 0.05-8.0% by weight of prenilamine, special cosmetic effects can be sought, for example deodorizing preventing pruritus, ulorrhoea, gingivitis, prevention of caries etc.

The effect upon gingivitis of the oral care agents which form a special, particularly advantageous group of the cosmetic preparations according to the invention have been investigated in exhaustive detail. A significant part of the normal microbial flora of the oral cavity falls within the spectrum of activity of prenilamine, and it was therefore to be expected that the regular use of the oral care agents according to the invention would result in a reduction of the number of microbes in the mouth. This is a result perse but it was further surprisingly found that with the regular use of the oral care agents according to the invention certain disorders of the oral cavity, for example ulorrhoea, gingivitis, caries and gomphiasis can be prevented or these disorders can be cured.

The toothpaste containing 0.9% by weight prenilamine lactate, prepared according to Example 10, was investigated with reference to a group consisting of 16 women and 7 men and having an average age of 37 years. The members of the group brushed their teeth twice a day for five weeks with the toothpaste according to the invention.

The control group consisting of 12 women and 7 men and having an average age of 34 years likewise brushed their teeth twice a day for five weeks with a toothpaste the composition of which correspond to the first, but did not contain prenilamine.

In the investigated group 8 persons suffered, and in the control group 6 persons suffered, from ulorrhoea which forms the first stage of gingivitis. In the group treated, the ulorrhoea disappeared after 8 days in the case of 3 persons, and after a further 6 days in the case of 4 persons. In the case of the remaining 1 person the ulorrhoea ceased only after 4 weeks of treatment. No charge occurred in the control group, and after the 5 weeks the 6 persons suffered from ulorrhoea without change.

The treatment with the toothpaste according to the invention consequently cured the ulorrhoea, probably in that the agent neutralizes the effect of the dentogingual plaque which causes gingivitis and caries, although it only slightly reduces the amount of plaque.

It was also found in the investigation described that the preparation did not damage the mucosa, but, on the contrary, the proportion of the hypercornified and superficial cells ensuring the normal defensibility of the mucosa increased slightly.

All the above leads to the conclusion that by using the oral care agents according to the invention it is possible to prevent gingivitis, to cure the ulorrhoea forming the first stage of the latter and to retard the occurrence of caries which is reckoned a national disease in many countries of the world False teeth are not discoloured by the oral care agents according to the invention.

The invention is described in greater detail with reference to the following Examples.

### Example 1

### Aerosol deodorant

Composition:

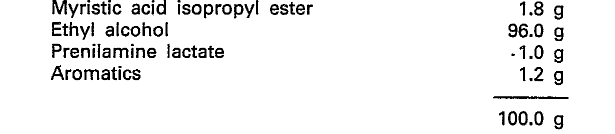

Propellent: Freon F 12@
The propellent end filling are in the ratio of 1:2.

The components are dissolved in the said quantity of ethyl alcohol, the solution is filtered and is then poured into aerosol spray canisters.

The preparation deodorizes and disinfects, and its reduces pruritus and the occurrence of allergic symptoms and prevents seborrhoea in greasy skin.

### Example 2

### Aerosol initimate deodorant

Composition:

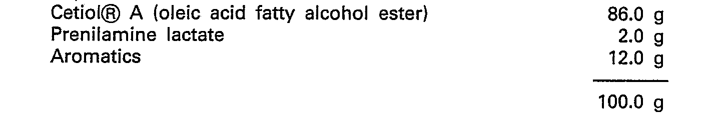

Propellent: Freon F 12@
The filling and propellent are in the ratio of 20:1.

The micronized prenilamine lactate is suspended in the quantity of Cetiol(g) indicated, and the suspension is mixed with the aromaics and then poured into aerosol canisters.

The preparation deodorizes and disinfects, and it reduces itching prevents the occurrence of allergic manifestations and can also be used to prevent fungal diseases and to combat discharge.

### Example 3

### Sun-tan cream (Aerosol)

Composition:

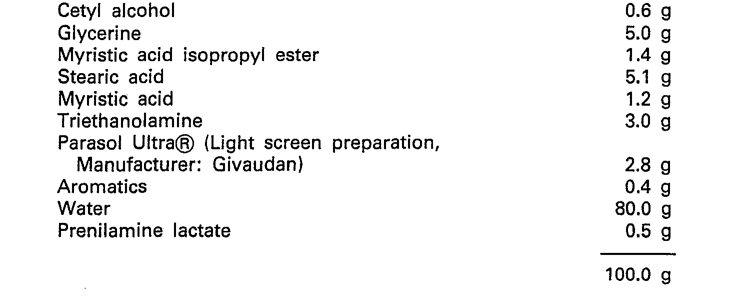

Propellent: Freon P 12/114®
The filling and propellent are in the ratio of 9:1.

Cetyl alcohol, myristic acid, stearic acid and myristic acid isopropyl ester are melted at approximately 70°C and homogenized with one another. At 70°C the prenilamine lactate, the glycerine and the triethanolamine are dissolved in the quantity of water indicated while being stirred, and the solution is slowly added to the homogeneous melt while being constantly stirred. The mixture obtained is cooled to 20°C, is reacted with the aromatic and the light screen preparation, and is finally poured into aerosol canisters.

In addition to its general cosmetic effect and the light-screening effect sought, the preparation reduces pruritus, inhibits the development of allergic symptoms and has a deodorizing effect.

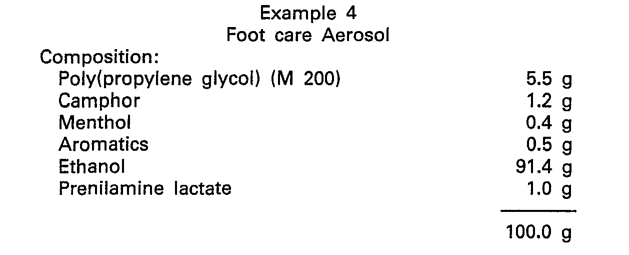

Propellent: Freon F 12@
The ratio of filling to propellent amounts to 2:1.

Prenilamine lactate, camphor and menthol are dissolved in the quantity of ethanol indicated and the poly(propylene glycol) and the aromatics are added to the solution. The liquid is filtered and is then poured into aerosol canisters.

The preparation inhibits the occurrence of fungus on the skin, deodorizes, disinfects and reduces the itchiness of the skin.

### Example 5

### Oral spray

Composition:

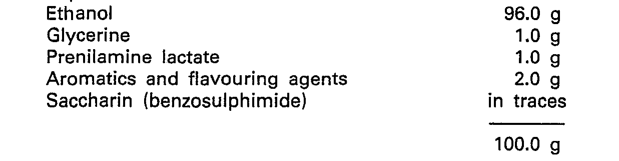

Propellent: Freon F 12@
The ratio of filling to propellent amounts to 1:1.

The components are dissolved at room temperature in the quantity of alcohol indicated. The solution is filtered and poured into aerosol canisters.

The preparation is suitable for deodorizing and disinfecting the oral cavity. It is used to prevent stomatitis, ulorrhoea, gingivitis and gomphiasis of the teeth. In addition, the preparation prevents the occurrence of caries.

### Example 6

### Aerosol

Composition:

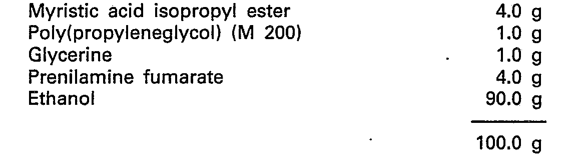

Propellent: Freon P 114@
The filling and propellent are in the ratio of 1:1.

The components are dissolved at room temperature in the quantity of ethanol indicated, and the solution is then filtered and poured into aerosol canisters.

The preparation offers protection against skin allergies, deodorizes, disinfects, is suitable for the case of scaly and greasy scalps, prevents the occurrence of fungus, reduces pruritus and combats the symptoms of urticaria.

### Example 7

### Hair tonic for greasy hair

### Composition:

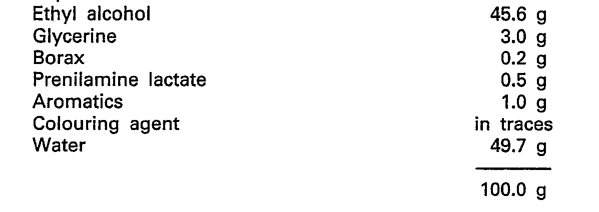

The components are dissolved in the quantity of alcohol indicated, the solution is filtered and is then poured out.

The preparation is suitable for treating scaly scalps, regenerating the hair, reducing the itching or the scalp, and preventing the occurrence of fungus and allergies. In addition, it prevents the static charging of the hair, improves its combability, normalizes the hair pigmentation and cures inflammation of the scalp.

### Example 8

### Toilet soap

### Composition:

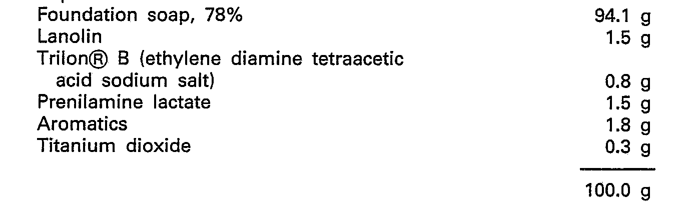

The components are homogenized, pilled, pressed into a billet and then the toilet soap is pressed into impact moulds. The soap can also be used by persons allergic to soap, without allergy occurring. The soap deodorizes, disinfects and reduces pruritus. In addition, it prevents the occurrence of fungus and other infections of the skin.

### Example 9

### Toothpaste

### Composition:

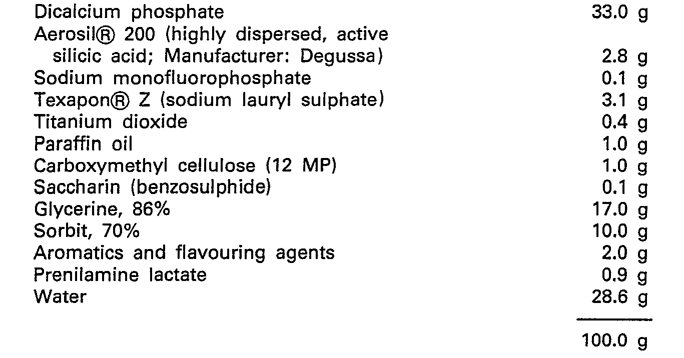

The saccharin and the prenilamine lactate are dissolved in a quarter of the quantity of water indicated. The solution is heated to 80°C, is reacted with the carboxymethyl cellulose and is then cooled to 25°C over approximately 112 hours while being constantly stirred. The sodium monofluorophosphate is dissolved in the remainder of the water and is then mixed with the solution first prepared. The glycerine, the paraffin oil, the sorbit, the dicalcium phosphate, the Aerosil@ and Texapon@, the titanium dioxide and finally the aromatics are added to the thick gel. The mixture is homogenized until it is free from lumps, is then degassed in a vacuum and is finally poured into tubes.

The toothpaste is used to prevent stomatitis and gingivitis, and in addition to disinfect and deodorize, and it also prevents ulorrhoea, caries and gomphiasis of the teeth.

### Example 10

### Shampoo

### Composition:

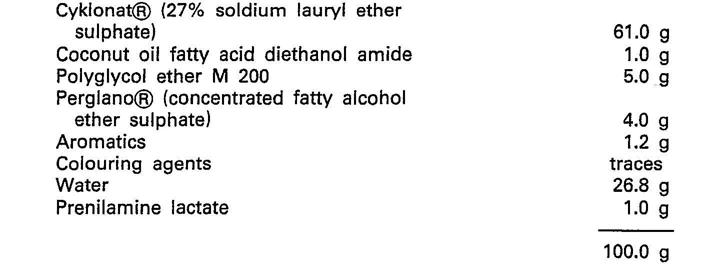

The prenilamine, the Cyklonat@, the polyglycol ether and the coconut oil fatty acid diethanol amide are dissolved at 40°C in two thirds of the indicated quantity of water, and the suspension of the Perglano concentrate in the remaining quantity of water is added to the solution while being constantly stirred. The mixture is allowed to cool to 20°C while being stirred, and the aromatics and the colouring agents are then added thereto. The product is poured out.

The preparation can be used to combat allergies of the scalp and the formation of dandruff and to prevent pruritis. It disinfects and prevents fungal disorders, and, in addition, it regenerates the hair and prevents it from being statically charged. The medium promotes the growth of hair and pigmentation and prevents inflammatory disorders of sensitive and greasy scalps.

### Example 11

### Foam bath

### Composition:

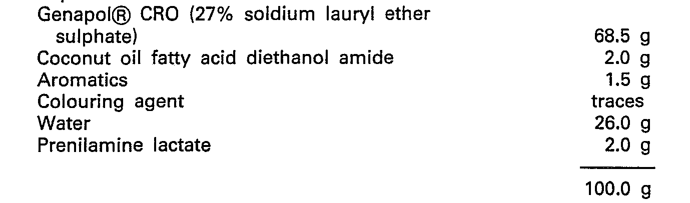

The preparation is produced in the manner described in Example 11 [sic]. The foam bath combats pruritus, skin-allergy manifestations, acts as a disinfectant and prevents fungal disorders.

### Example 12

### Shaving cream (rapid)

### Composition:

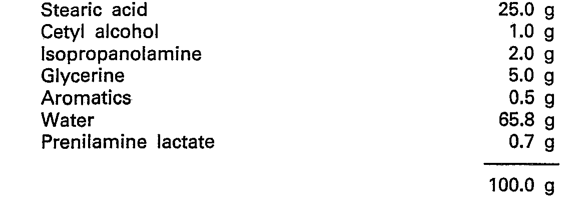

The preparation is produced from two stages. One phase is stearic acid, which is heated to 70°C. The second (aqueous) phase is produced by dissolving the cetyl alcohol, isopropanolamine, glycerine and prenilamine lactate in water, the temperature being increased to 70°C with constant stirring during the dissolution procedure. The second phase is added to the first phase at the same temperature with constant stirring. After gradual cooling to 30°C the aromatics are added, and then the product is poured out.

The shaving cream acts as a disinfectant, prevents fungal disorders, pruritus and allergic manifestations, and is pleasant to use.

### Example 13

### Milk cream

### Composition:

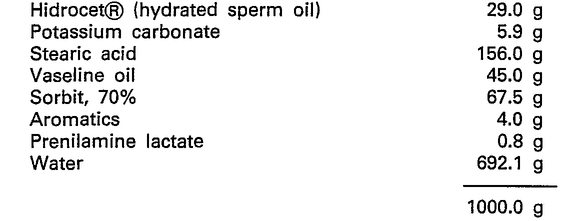

The preparation is made from two phases. One phase (fatty phase) is prepared from the stearic acid which is melted at 80°C and is homogenized with the Hidrocet@ and the Vaseline oil. The aqueous phase is produced by dissolving the potassium carbonate and the sorbit solution in water. The aqueous phase is added to the fatty phase at 80°C, and after cooling to 60°C the prenilamine lactate is added, and after cooling to 30°C the aromatic is added. The product is poured out.

The milk cream is suitable for the care of sensitive skin. It prevents the formation of skin-allergy manifestations and reduces pruritus.

### Example 14

### Hand protection cream

### Composition:

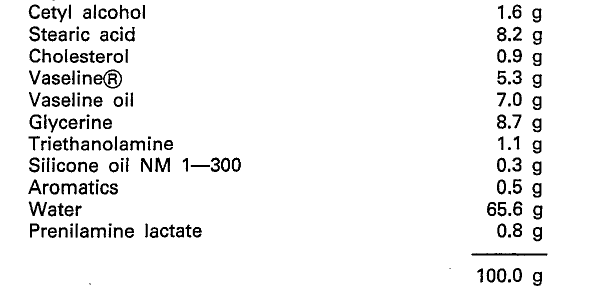

The preparation is produced from two phases. The first phase is the melt of vaseline oil, Vaseline@, cetyl alcohol, stearic acid and cholesterol, prepared at 80°C. The second phase is produced by dissolving the triethanolamine, glycerine and prenilamine lactate or water and homogenizing the solution at 80°C. The second phase is added to the first phase at the same temperature. After cooling to 30°C, the aromatics are added. The cream is homogenized, degassed in a vacuum and then poured out.

The cream is suitable for calming hypersensitive skin and for preventing skin allergies and pruritus, and in addition it acts as a disinfectant.

### Example 15

### Hydrating cream

### Composition:

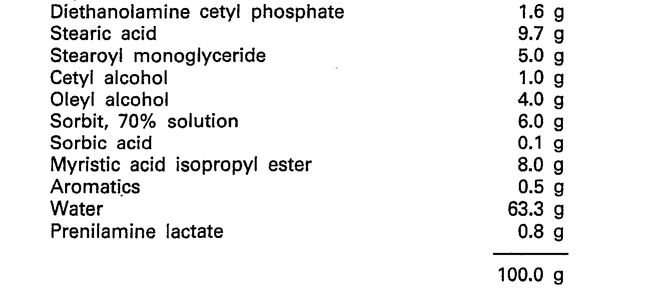

The preparation is produced from two phases. The first phase (fatty phase) is the mixture of myristic acid isopropyl ester, oleyl alcohol, diethanolamine cetyl phosphate, cetyl alcohol, stearin and stearyl monoglyceride, homogenized at 80°C. In order to produce the second (aqueous phase), the sorbit solution and the prenilamine lactate are homogenized in water at 80°C. The aqueous phase is added to the fatty phase, after cooling to 30°C the aromatics are added, and then the cream obtained in homogenized, degassed in a vacuum and is finally poured out.

In addition to its hydrating effect the cream inhibits the formation of skin allergies and fungal disorders and relieves pruritus.

### Example 16

### Baby cream

### Composition:

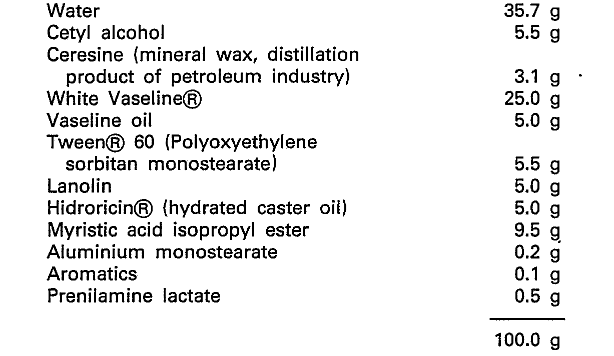

The preparation is produced from two phases. The fatty phase consists of the following components: aluminium monostearate, ceresine, Hidroricin@, cetyl alcohol, lanolin, myristic acid isopropyl ester, white Vaseline@, vaseline oil and Tween@. The components are homogenized together at 80°C. The aqueous phase is formed by the solution of the prenilamine lactate in water. The aqueous solution is added to the fatty phase in the heat while being stirred. After homogenization the mixture is cooled to 30°C, provided with the aromatics, homogenized once again, degassed in a vacuum and finally poured out.

The cream is suitable for hypersensitive skin, and for baby care. It acts as a disinfectant, relieves pruritus, erythema and at the same time acts as a deodorant.

It reduces the tendency to oral mucor.

### Example 17

### Grease

### Composition:

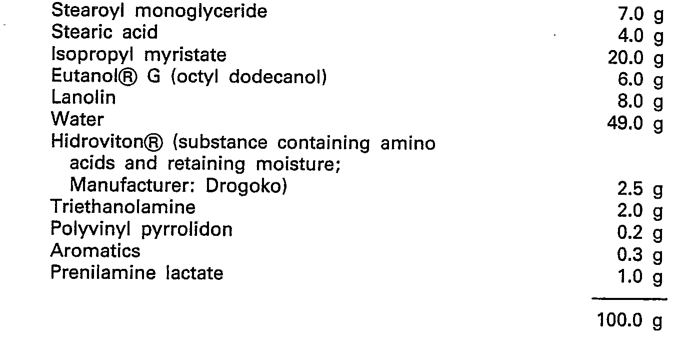

The components of the fatty phase are mixed together at 80°C: stearoyl monoglyceride, stearic acid, Euthanol G@, lanolin and isopropyl myristate.

The components of the aqueous phase―water Hidroviton@, triethanolamine, polyvinyl pyrrolidon and prenilamine lactate-are likewise homogenized together at 80°C. The aqueous phase is added to the fatty phase in the heat while being stirred. After the mixture has cooled to 30°C, the aromatics are added, homogenization is performed again, the cream is degassed in a vacuum and is finally poured out.

The cream has an anti-allergic action and prevents fungal disorders. It relieves hypersensitivity of the skin and pruritus and it has, in addition, a deodorizing effect.

### Example 18

### Mouthwash

### Composition:

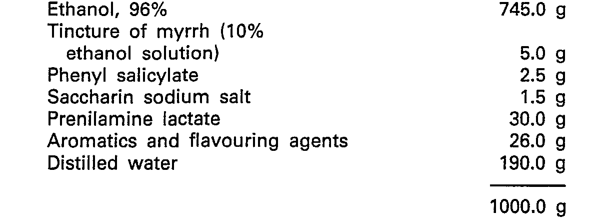

The prenilamine lactate is dissolved at room temperature in the indicated quantity of ethanol, the tincture of myrrh is added, and phenyl salicylate and aromatics are added, the saccharin sodium salt is dissolved in water. The aqueous solution is added to the alcohol solution while being stirred, the liquid is filtered and then poured out.

The mouthwash is suitable for preventing stomatitis and gingivitis, it deodorizes and disinfects the oral cavity and prevents ulorrhoea.

## Claims

1. The use of 3,3-diphenylpropyl-1-methylphenethylamine or one of its acid addition salts in cosmetic preparations.

2. The use of 3,3-diphenylpropyl-1-methylphenethylamine lactate according to Claim 1.

3. The use of 3,3-diphenylpropyl-1-methylphenethylamine fumarate according to Claim 1.

4. The use of 0.05-8.0% by weight of 3,3-diphenylpropyl-1-methylphenethylamine or 3,3-diphenylpropyl-1-methylphenethylamine acid addition salt, 20-99% by weight of solvent, 0-70% by weight of washing active substances and/or emulsifying agents, 0-20% by weight of cosmetic additives and 0-35% by weight of fillers according to Claim 1, which where appropriate are poured into aerosol canisters, a propellent also being present in a weight ratio between the preparation to be sprayed and the propellent of 1:1 to 20:1.

5. The use of 3,3-diphenylpropyl-1-methylphenethylamine or the acid addition salt thereof according to Claim 1 in mouthwashes.

6. A toothpaste, characterized in that it contains 0.05-8.0% by weight of 3,3-diphenylpropyl-1-methylphenethylamine or the acid addition salt thereof and the components usual for toothpastes.

7. A deodorant, characterized in that it contains 0.05-8.0% by weight of 3,3-diphenylpropyl-1-methylphenethylamine or the acid addition salt thereof and the components usual for deodorants.

8. A method of preparing cosmetic preparations according to Claim 4, characterized in that 0.05-8.0% by weight of 3,3-diphenylpropyl-1-methylphenethylamine or the acid addition salt thereof is dissolved in a solvent or mixture of solvents, where desired up to a solvent proportion of 20-99% by weight of additional solvent, the solution is mixed with altogether 0-70% by weight of washing active substances and/or emulsifying agents, 0-20% by weight of cosmetic additives and 0-33% by weight of fillers and is poured into the packaging units or containers usual for cosmetic products.

## Patentansprüche

1. Verwendung von 3,3-Diphenylpropyl-1-methylphenäthylamin oder eines seiner Säureadditionssalze in kosmetischen Präparaten.

2. Verwendung von 3,3-Diphenylpropyl-1-methylphenäthylaminlactat nach Anspruch 1.

3. Verwendung von 3,3-Diphenylpropyl-1-methylphenäthylaminfumarat nach Anspruch 1.

4. Verwendung von 0,05-8,0 Gew.-% 3,3-Diphenylpropyl-1-methylphenäthylamin oder 3,3-Diphenylpropyl-1-methylphenäthylamin-Säureadditionssalz, 20-99 Gew.-% Lösungsmittel, 0-70 Gew.-% waschaktiver Substanzen und/oder Emulgiermittel, 0-20 Gew.-% kosmetischer Zusatzstoffe und 0-35 Gew.-% Füllstoffe nach Anspruch 1, die gegebenenfalls in Aerosolbehälter gefüllt sind, wobei auch ein Treibgas vorliegt, in einem Gewichtsverhältnis zwischen zu zerstäubendem Präparat und Treibgas von 1:1 bis 20:1.

5. Verwendung von 3,3-Diphenylpropyl-1-methylphenäthylamin oder dessen Säureadditionssalz nach Anspruch 1 in Mundwässern.

6. Zahnpasta, dadurch gekennzeichnet, daß sie 0,05―8,0 Gew.-% 3,3-Diphenylpropyl-1-methylphenäthylamin oder dessen Säureadditionssalz und die für Zahnpasten üblichen Komponenten enthält.

7. Desodorant, dadurch gekennzeichnet, daß es 0,05-8,0 Gew.-% 3,3-Diphenylpropyl-1-methylphenäthylamin oder dessen Säureadditionssalz und die für Desodorants üblichen Komponenten enthält.

8. Verfahren zur Herstellung von kosmetischen Präparaten gemäß Ansrpuch 4, dadurch gekennzeichnet, daß man 0,05-8,0 Gew.-% 3,3-Diphenylpropyl-1-methylphenäthylamin oder dessen Säureadditionssalz in einem Lösungsmittel oder Lösungsmittelgemisch löst, gewünschtenfalls bis zu einem Lösungsmittelanteil von 20-99 Gew.-% weiteres Lösungsmittel zugibt, die Lösung mit insgesamt 0-70 Gew.-% waschaktiven Substanzen und/oder Emulgiermitteln, 0-20 Gew.-% kosmetischen Zusatzstoffen und 0-35 Gew.-% Füllstoffen vermischt und in die für kosmetische Produkte üblichen Verpackungseinheiten oder Gefäße füllt.

## Revendications

1. Utilisation de la 3,3-diphénylpropyl-1-méthylphénéthylamine ou de l'un de ses sels formés par addition avec des acides dans des produits cosmétiques.

2. Utilisation du lactate de 3,3-diphénylpropyl-1-méthylphénéthylamine selon la revendication 1.

3. Utilisation du fumarate de 3,3-diphénylpropyl-1-méthylphénéthylamine selon la revendication 1.

4. Utilisation de 0,05 à 8,0% en poids de 3,3-diphénylpropyl-1-méthylphénéthylamine ou d'un sel de 3,3-diphénylpropyl-1-méthylphénéthylamine formé par addition avec un acide, 20 à 99% en poids de solvant, 0 à 70% en poids de substance détergente et/ou d'agents émulsionnants, 0 à 20% en poids d'additifs cosmétiques et 0 à 35% en poids de matières de charge selon la revendication 1, éventuellement introduits dans des récipients aérosols, auquel cas il y a encore un gaz propulseur et les proportions relatives en poids entre le produit à atomiser et le gaz propulseur vont ds 1:1 à 20:1.

5. Utilisation de la 3,3-diphénylpropyl-1-méthylphénéthylamine ou de l'un de ses sels formés par addition avec un acide selon la revendication 1, dans des lotions pour les soins de la bouche.

6. Pâte dentifrice, caractérisée en ce qu'elle contient de 0,05 à 8,0% en poids de 3,3-diphénylpropyl-1-méthylphénéthylamine ou de l'un de ses sels formés par addition avec un acide et les composants usuels dans les pâtes dentifrices.

7. Désodorisant corporel, caractérisé en ce qu'il contient de 0,05 à 8,0% en poids de 3,3- diphénylpropyl-1-méthylphénéthylamine ou de l'un de ses sels formés par addition avec un acide et les composants usuels pour les désodorisants corporels.

8. Procédé de préparation de produits cosmétiques selon la revendications 4, caractérisé en ce que l'on dissout de 0,05 à 8,0% en poids de 3,3-diphénylpropyl-1-méthylphénéthylamine ou de l'un de ses sels formés par addition avec un acide dans un solvant ou mélange solvant, on ajoute si on le désire des compléments de solvant jusqu'à une proportion totale de solvant de 20 à 99% en poids, un mélange la solution avec 0 à 70% en poids au total de substances détergentes et/ou d'agents émulsionnants, 0 à 20% en poids d'additifs cosmétiques et 0 à 35% en poids de matières de charge et on introduit dans les unités d'emballage ou récipients usuels pour les produits cosmétiques.
